# EUROPEAN PATENT APPLICATION

(11) **EP 1 498 087 A2**
(43) Date of publication of application: **19.01.2005**
(21) Application number: 04254317.3
(22) Date of filing: 19.07.2004
(51) Int. Cl.: A61F 2/14

(54) **Artificial cornea**

(30) Priority: 17.07.2003 GB 0316745
(71) Applicant: I.O International Ltd., Croydon, Surrey CR2 8LE (GB)
(72) Inventor: Darougar, Sohrab, Croydon Surrey CR2 8LE (GB); Darougar, Dayshad, Croydon Surrey CR2 8LE (GB)
(74) Representative: Kirkham, Nicholas Andrew

(57) **Abstract**

The present invention relates to a keratoprosthesis or artificial cornea which is designed as a light weight disc resembling a human cornea. The keratoprosthesis is maintained in the eye after surgery and is preferably adapted to reduce complications after surgery such as infection.

## Description

The present invention relates to a keratoprosthesis or artificial cornea, and a method for implanting a keratoprosthesis.

Keratoprosthesis are referred to as an artificial cornea as the keratoprosthesis replaces the cornea of the patient, for example, when this is damaged by infection, trauma or otherwise. Although the visual part of the keratoprosthesis is referred to as a "lens", a keratoprosthesis should not be confused with an intra-ocular lens (or artificial lens), as there are no functional or physical similarities between the two devices. In particular, the optical part of each device will mirror the optical properties of a natural cornea/lens and so have totally different optical properties. The intro-ocular lens is designed to replace the opaque lens of a patient due to cataract and resides within the eye. This is distinct from the keratoprosthesis which resides in the front part of the eye.

Corneal blindness is a major health, social and economic problem worldwide. It accounts for up to 5% of all blindness in the developed countries and for up to 50% in the developing countries. It is estimated that at least 10 million people suffer from bilateral corneal blindness and probably a larger number from unilateral corneal blindness.

There are a variety of causes responsible for corneal blindness. In the developed countries, the common causes are herpes simplex virus corneal infection, trauma, chemical and metal burn, cicatricial pemphigoid, Stevens-Johnson syndrome, Rosacea keratitis, corneal dystrophy, dry eye and failed keratoplasty. In developing countries, corneal infections such as trachoma, bacterial and fungal corneal ulcer and abscess, herpes simplex virus corneal infection, onchocerciasis (river blindness) and a post-measles keratitis are the main causes of corneal blindness. A number of these diseases cause severe corneal scarring and vascularization, uneven corneal thickness and damage to the anterior segment of the eye.

In developed countries, keratoplasty has been successful in restoring vision in patients with uncomplicated corneal scarring, while, in patients with severe corneal scarring and vascularization and in those with damaged anterior segment, the results of keratoplasty have been rather poor. In the developing countries, keratoplasty is not frequently performed because of the scarcity of donor corneas.

In developed countries, of an estimated 2 million blind people (0.2% of the population) 100,000 (5%) are blind because of corneal diseases. The incidence of corneal blindness is estimated at about 5000 per annum. In the USA over 50,000 corneal transplantations are being carried out annually. In developing countries it is estimated that 10 million people suffer from blindness because of corneal diseases. The incidence of corneal blindness is estimated at about 450,000 cases per annum.

The concept of using a keratoprosthesis or artificial cornea in patients with corneal blindness was suggested as early as the 18th century. Early studies using glass, crystal and celluloid implants began in the 19th century. However, due to poor results and the advent of keratoplasty in the early 20th century, interest in artificial cornea diminished. Fifty years later, it was found that polymethylmethacrylate (PMMA) a synthetic polymer with low toxicity and good optical quality could be retained in the cornea of animals and humans for long periods without major adverse effect. Following these findings, interest in the development of artificial cornea revived.

Studies showed that the full penetrating cylinder of PMMA can restore vision but only for a short period, because of its very high extrusion rate. For example, see US-A-5 300 116 and US-A-4 593 715.

The retainability of these penetrating optical cylinders was improved by adding to it a supporting plate which was buried in the corneal stroma. For example, see US-A-5 458 819, US-A-6 106 552, US-A-6 254 637 and US-A-5 489 301.

The supporting plate wasthen modified using various anchoring materials such as tooth, cartilage, terylene, dacron, ceramic and metal. For example, see EP-A-0 956 834, US-A-5 713 956, US-A-4 581 030, US-A-5 843 185, US-A-5 836 313 and US-A-5 489 300.

However, due to further advances in the design of keratoprosthesis, such as 2-piece implants by Stone and Choyce, a 2-piece "bolt and nut" by Cardona and keratoprosthesis with flexible plate, the retainability of keratoprosthesis has improved. For example, see US-A-3 945 054, US-A-4 470 159, US-A-4 842 599, US-A-5 030 230, US-A-5 067 961, US-A-5 354 332, US-A-4 923 466, US-A-4 563 779, US-A-5 632 773 and US-A-4 586 929.

In 1991, a study was arranged to assess the suitability and effectiveness of a soft keratoprosthesis designed by Caldwell and Py in restoring sight in patients with corneal blindness in Karachi, Pakistan. The Caldwell-Py keratoprosthesis was made of polyurethane elastomers and consisted of a central transparent optical part and a peripheral white and porous skirt with extended arms attached to the skirt to fix the keratoprosthesis to the sclera. The keratoprosthesis was implanted in six eyes and results were followed for one year. Vision was improved from light perception to a level of 3/60 in two eyes, to counting fingers at two metres in two eyes, but deteriorated later and did not improve at all in the remaining two eyes, because of pre-operative retinal disfunction (unpublished report). The main adverse effects and complications were discolouration, dryness and erosion of the optical part and adhesion of cells and debris to its surface in all eyes; partial extrusion of keratoprosthesis in five eyes, because of poor binding of the skirt to surrounding tissues; moderate to severe intra-ocular infection in four eyes, closely associated with the extrusion of keratoprosthesis; and moderate to severe retroprosthetic membrane in four eyes, associated with moderate to severe damage to the anterior segment of the eye.

The present invention seeks to provide a keratoprosthesis which is retained in the eye without causing major complications and adverse effects commonly associated with available keratoprosthesis.

According to a first aspect of the present invention, there is provided a keratoprosthesis comprising a lens surrounded by a skirt, where the keratoprosthesis is a one-part construction with the thickness of the lens being greater than the thickness of the skirt.

The lens is generally circular with a diameter of between 5mm and 9mm, preferably about 7mm; and/or the skirt comprises a generally circular ring around the lens with the ring having a width of between 1mm and 3mm, preferably about 2mm. The overall construction is thus small and lightweight.

The lens preferably has a thickness of between 0.5mm and 2mm, preferably about 1mm; and/or the skirt has a thickness of between 0.1mm and 0.4mm, preferably about 0.3mm. The overall thickness of the construction is thus small compared to the presently available artificial corneas.

The keratoprosthesis skirt advantageously includes a plurality of large holes of about 1 mm diameter; and/or the skirt includes a plurality of small holes of about 0.3mm diameter. The large holes are particularly useful for suturing. The large and small holes allow the tissues of the eye to grow therebetween and fluids to cross the plane of the implant.

The keratoprosthesis is advantageously formed from a biocompatible material selected from the group consisting of: silicon elastomers; PMMA including modified and/or crosslinked PMMA; HEMA; polyHEMA; mixtures of the aforementioned compounds.

The skirt is preferably coated with a polymer which promotes the binding of the keratoprosthesis in the eye which improves retainability of the implant.

Advantageous or preferred features of the keratoprosthesis are given in claims 2 to 6. The advantages of which will be clear from the following description.

According to a second aspect of the present invention, there is provided a kit comprising the keratoprosthesis according to the first aspect of the invention and as defined in claims 7 and 8 and a suture assembly where only two types of suture is required to hold the keratoprosthesis in place when inserted in the eye of a patient. The special suture can advantageously be used to secure other devices.

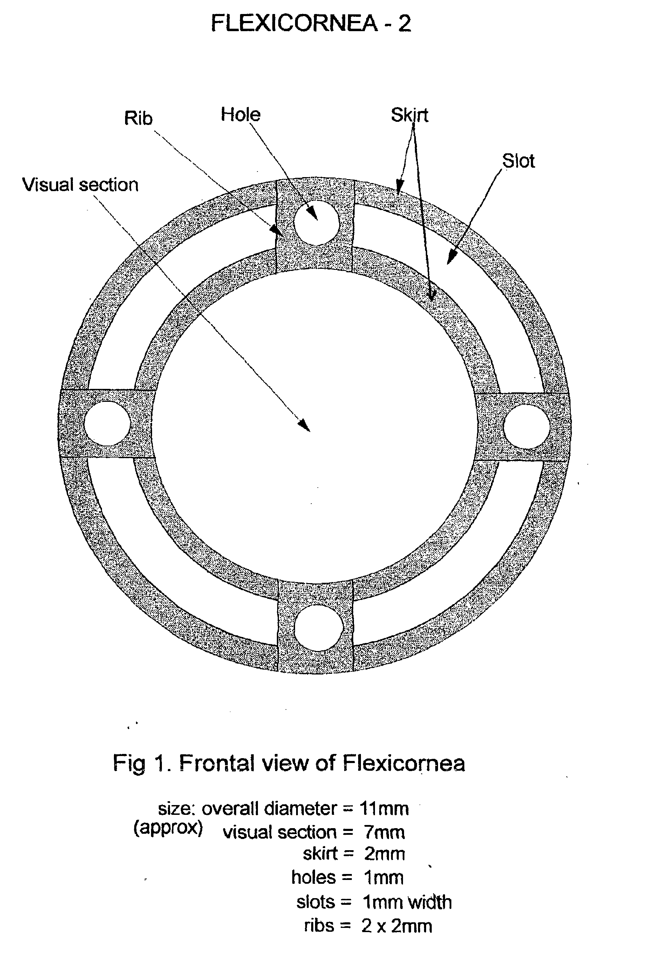

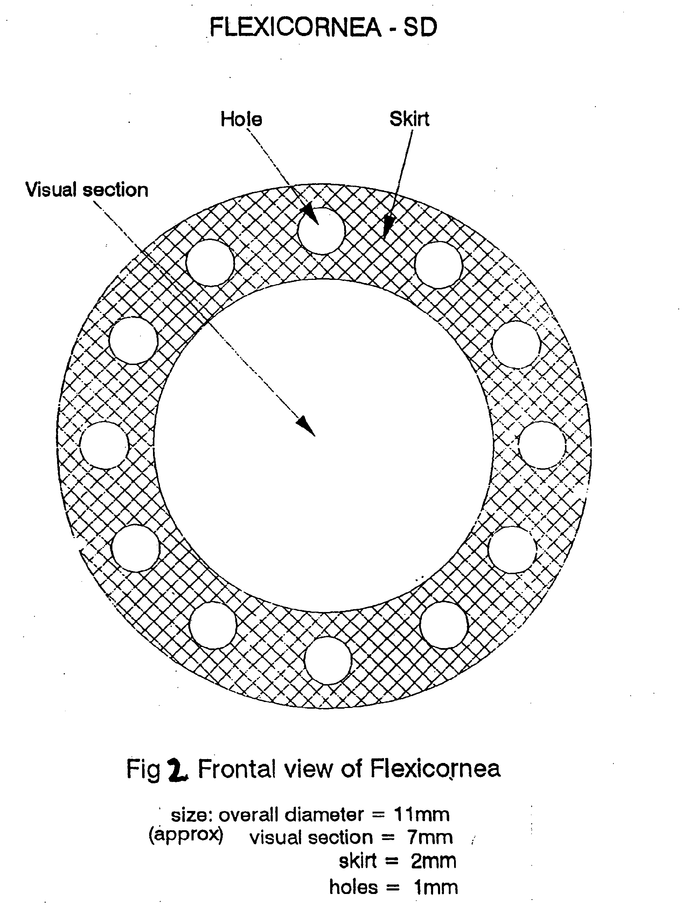

In a preferred embodiment the keratoprosthesis is a one-piece convex disc resembling the human cornea with a refractive power of 45 to 48 dioptre or higher. Advantageously the keratoprosthesis consists of a full thickness transparent central visual section with smooth surfaces (Figs 1 and 2) and a thinner net-type peripheral section or skirt with additional large holes.

According to a third aspect of the invention there is provided a specific corneal shield as defined in claim 9.

The keratoprosthesis of the present invention may be used as a method of installing a keratoprosthesis comprising the steps of:
(a) anaesthetizing the eye according to the standard procedure for intra-ocular operation;
(b) trephining the recipient cornea e.g. to the depth of 0.5mm approximately;
(c) dissecting the peripheral cornea into two flaps (lamella) until reaching the limbus;
(d) placing the keratoprosthesis near the cornea, passing the sutures between the two dissected flaps of the cornea and bringing them out just behind the limbus;
(e) removing the cornea button e.g. by cutting it with a pair of scissors;
(f) examining the anterior chamber and removing all debris and damaged tissues including the iris, lens, capsule and synechiae, optionally insert intra-ocular lens or artificial iris if required;
(g) positioning the keratoprosthesis in place of the corneal button, inserting the skirt between the two flaps of the recipient peripheral cornea and tightening the sutures;
(h) making sure that the keratoprosthesis is in position and that the skirt is firmly and correctly positioned between the two flaps of the cornea;
(i) tightening the sutures and knotting them over the sclera;
(j) optionally using surgical glue to initially attach the corneal flaps to the skirt until a strong binding between the skirt and corneal flaps develops.
It will be apparent that this results in a much simpler installation than current implantation procedures.

The invention will now be described with reference to the accompanying drawings, in which:
Fig. 1 depicts a front view of a keratoprosthesis according to a first preferred embodiment of the present invention;
Fig. 2 depicts a front view of a keratoprosthesis according to a second preferred embodiment of the present invention;
Fig. 3 depicts a cross-sectional side view of Fig. 1 or Fig. 2;

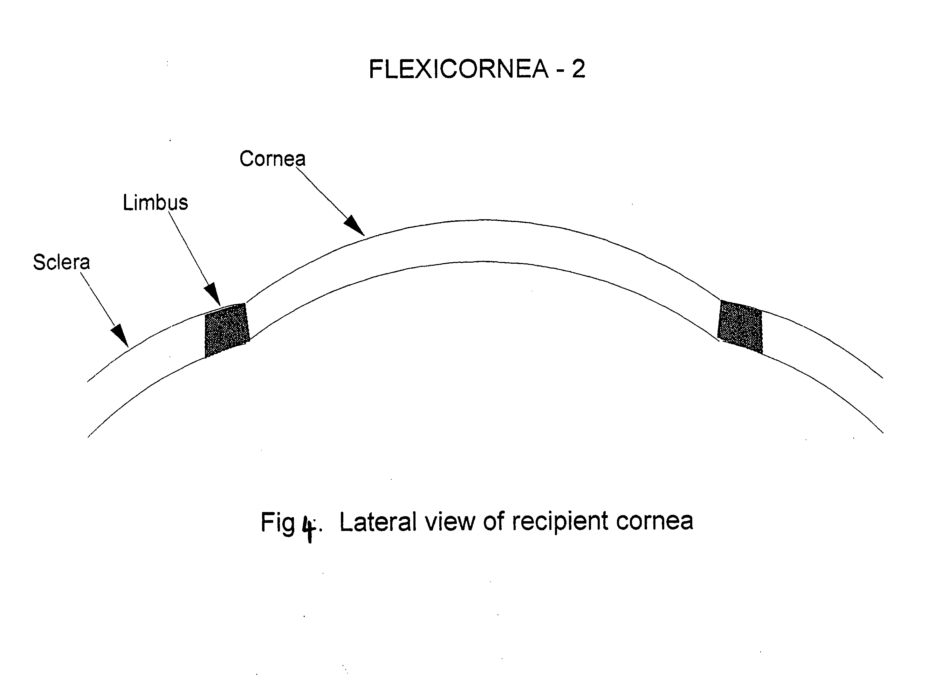

Fig. 4 depicts a cross-sectional side view of a patients cornea;
Fig. 5 depicts a patients cornea showing incision made during operation according to the method of the present invention;
Fig. 6A shows the view of Fig. 5 during a further operational step;
Fig. 6B shows the view of Fig. 6A during a further operational step;
Fig. 7 shows the view of Fig. 6A during a further operational step adopting the keratoprosthesis of Fig. 1(8A) and Fig. 2(8B);
Fig. 8A shows a front view of Fig. 7 adopting the keratoprothesis of Fig. 1;
Fig. 8B shows a frint view of Fig. 7 adopting the keratoprothesis of Fig. 2;
Fig. 9 shows the view of Fig. 7 during a further operational step;
Fig. 10 shows the view of Fig.8A with sutures tied;
Fig. 11 shows the view of Fig. 8B with the sutures tied.

In the illustrated examples, the drawings apply to both the first and second preferred embodiments of the keratoprosthesis except where specifically indicated.

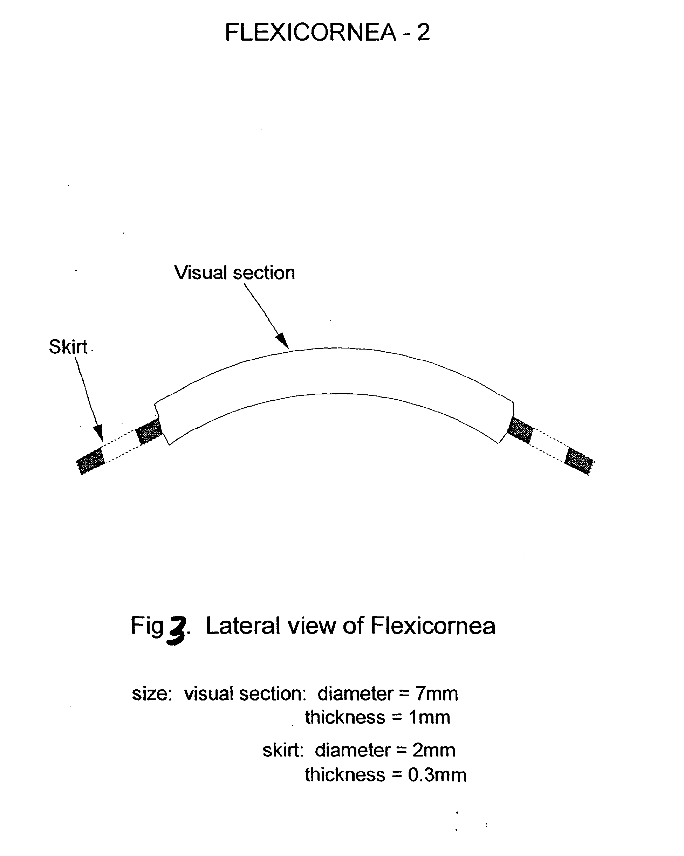

In the illustrated examples the overall diameter of keratoprosthesis is 11 m (Fig. 1). The visual section has a diameter of 7mm and a thickness of 1 mm (Fig. 1, 2 and 3). The width of the skirt is 2mm with a thickness of 0.3mm approximately (Fig. 1, 2 and 3). The diameter of large holes in the skirt is 1 mm approximately (Fig. 2). The slots have a width of 1 mm approx and a length of about 6/7mm.

The keratoprosthesis (also referred to as a flexicornea) will advantageously be made from a single polymer with the following features: highly transparent; semiflexible; highly tensile; non-erodible; non-binding; durable with no physical changes after long-term exposure to tears, aqueous, air and extreme climatic conditions; non-toxic and bio-compatible with human tissues. Silicon elastomers, PMMA with some modifications and cross-linking (to make it flexible), HEMA, PolyHEMA and other polymers used for making flexible intra-ocular lens may be suitable for the production of the keratoprosthesis.

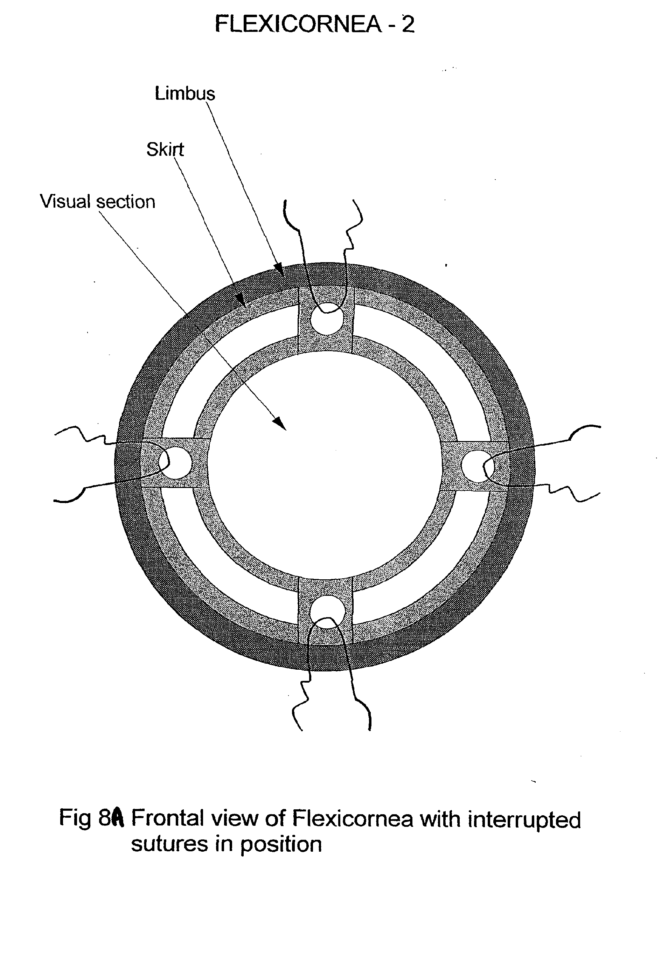

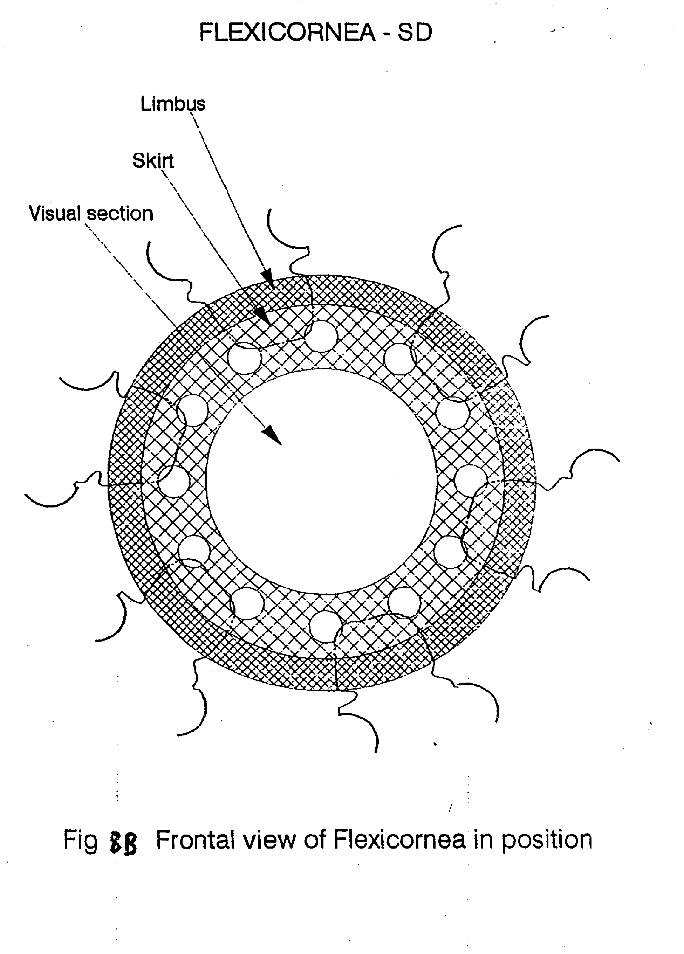

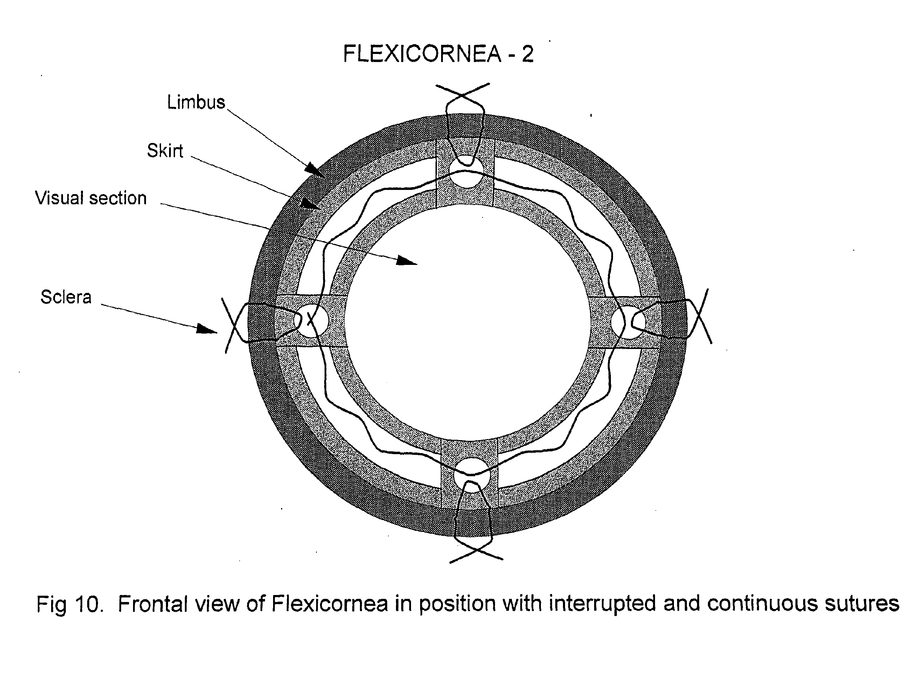

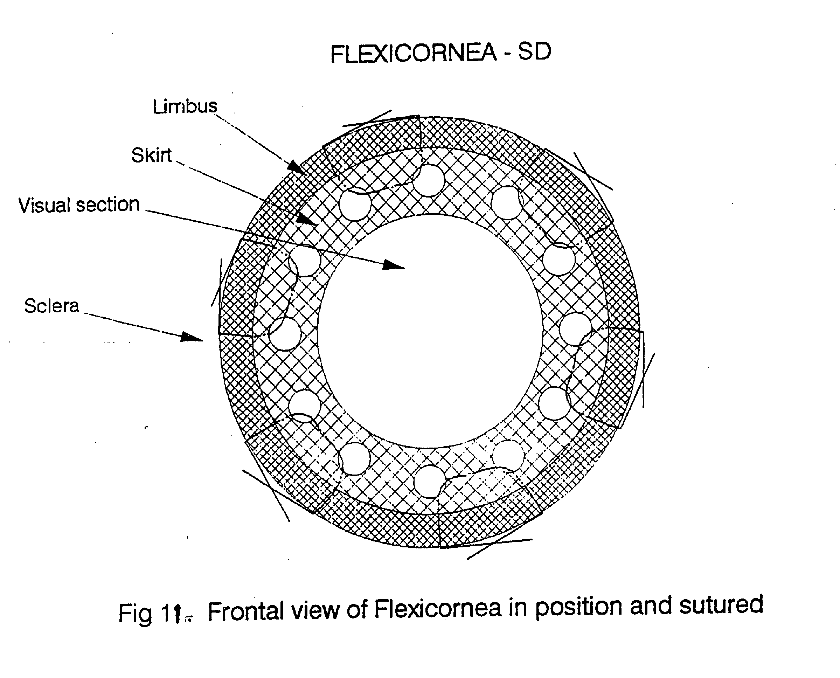

The skirt will be coated with one or more layers of a durable and non-erodible polymer with strong affinity to binding tissues, proteins and fibrin. Ready made sutures will attach to the keratoprosthesis (Fig. 8A, 8B, 10 and 11).

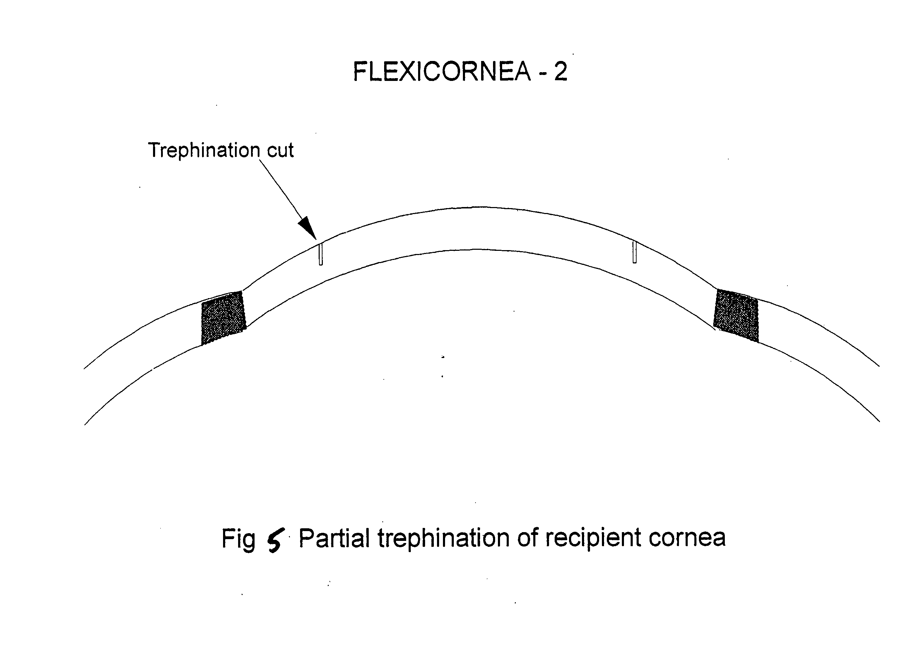

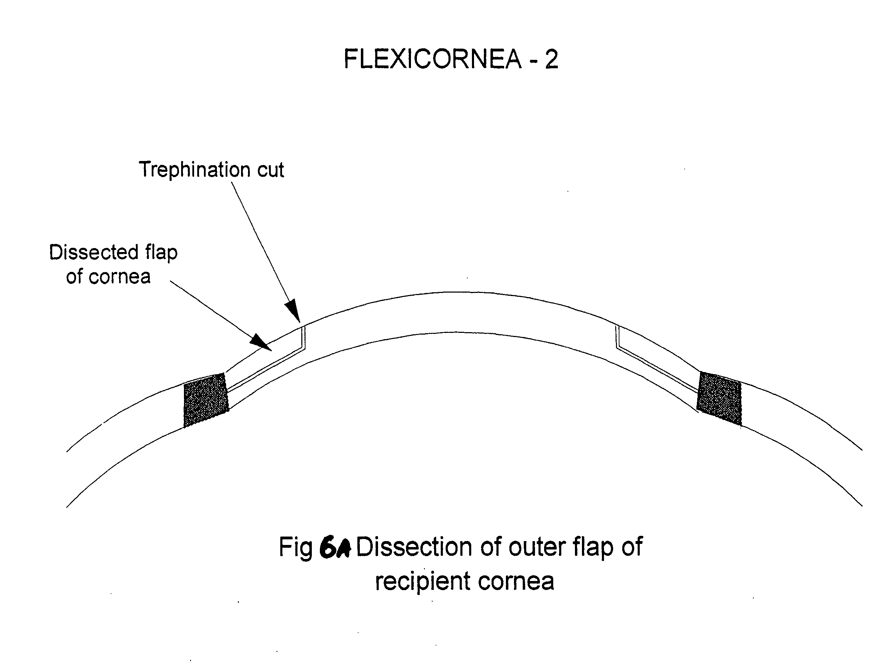

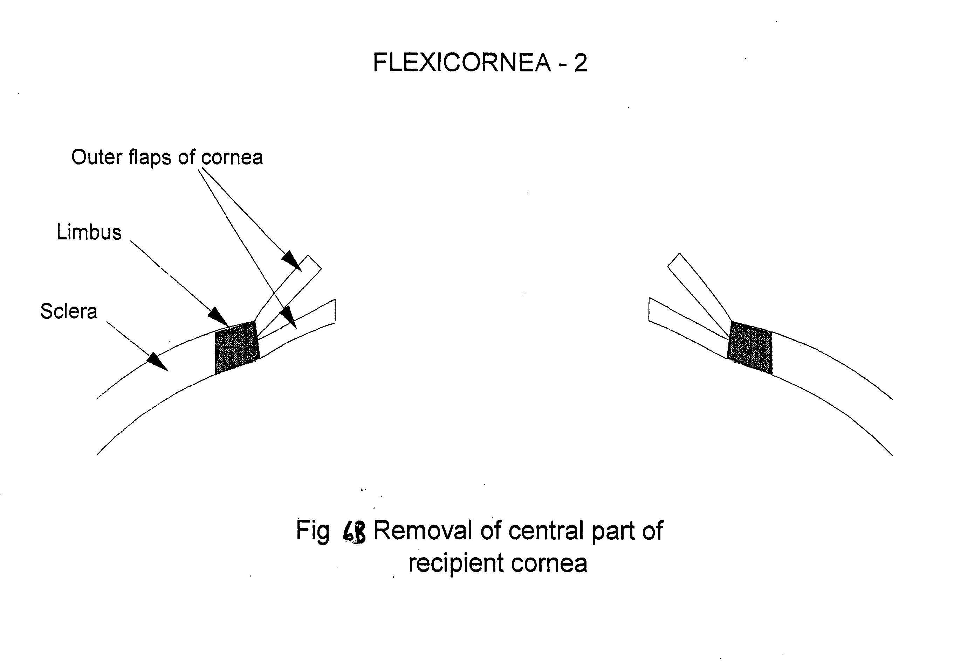

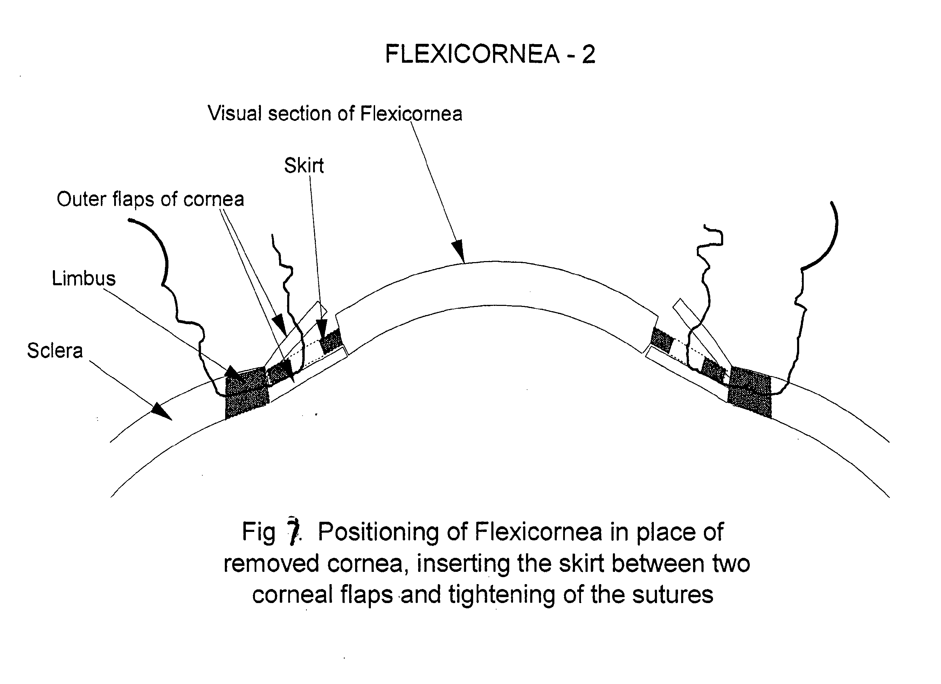

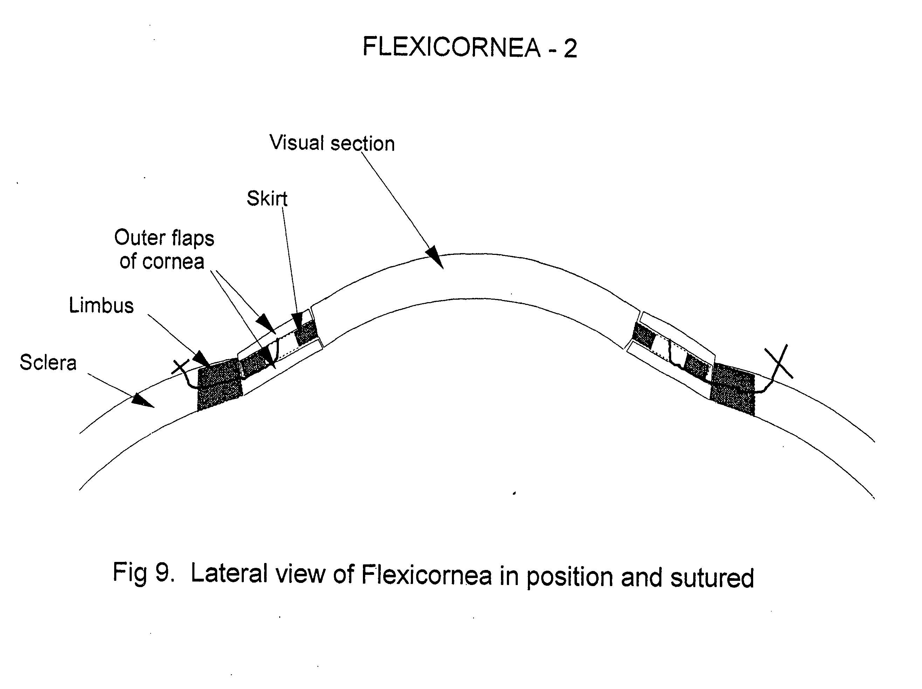

The operation to install the keratoprosthesis is simple, non-traumatic and similar to the keratoplasty operation. The main stages of the operation are as follows:
- Anaesthetize the eye according to the standard procedure for intra-ocular operation;
- Trephine the recipient cornea to the depth of 0.5mm approximately (Fig. 5);
- Dissect the peripheral cornea into two flaps (lamella) until reaching the limbus (Fig. 6A);
- Remove the cornea button by cutting, e.g. with a pair of scissors (Fig. 6B);
- Examine the anterior chamber and remove all debris and damaged tissues including the iris, lens, capsule and synechiae. Explore the possibility of inserting an intra-ocular lens;
- Position the keratoprosthesis in place of the corneal button, insert the skirt between the two flaps of the recipient peripheral cornea and tighten the sutures (Fig. 9). Pass sutures between the two dissected flaps of the cornea and bring them out just behind the limbus (Fig. 7);
- Make sure that the keratoprosthesis is in position and that the skirt is firmly and correctly positioned between the two flaps of the cornea;
- Tighten the sutures and knot them over the sclera; It would be beneficial if a surgical glue can be used to initially attach the corneal flaps to the skirt until a strong binding between the skirt and corneal flaps develop;
- Cover the cornea with a collagen shield or therapeutic contact lens releasing antibiotics and close the eye.

The invention in its preferred embodiment has a number of preferred features which are:
- the thin lightweight disc type keratoprosthesis is radically different from the commonly used cylinder-type keratoprosthesis which are bulky, heavy and intrusive;
- the one-piece keratoprosthesis does not need complicated and expensive manufacturing processes required for producing the commonly used two-piece keratoprosthesis;
- the flexibility of keratoprosthesis makes it less prone to scratches and erosion and it may allow the use of conventional tonometres for measuring intra-ocular pressure;
- the thin skirt with a net-type structure, large holes and slots and coating with a binding polymer has the following advantages;
- insertion of a thin skirt between flaps of the peripheral cornea is very easy and practical;
- it has been shown that cornea tolerate and retain a thin prosthesis much better than a thick prosthesis;
- the coating of the skirt will provide a basis for strong and durable bonding between the skirt and corneal flaps.
These features may be used in conjunction with one another or alone.

The coating concept is very well known and much used in microbiology, where microtitre plates are being coated with relevant polymers to either enhance or prohibit deposition of proteins and growth of cells on the microtitre plate. The coating is also used to deliver active ingredients, e.g. to prevent complications.

Fig. 1 shows the particularly preferred design of the keratoprosthesis of the present invention. This has a lens section coated as hereinafter described and a slit also coated as hereinafter described. The skirt is in the form of two rings joined by four ribs thus defining four large slots. Each rib has a hole.

The slots and holes shown in Fig. 1 or small and large holes in the skirt shown in Fig. 2 will allow the corneal and scar tissues to grow through the skirt and produce a strong fusion with the tissues of the opposite flap. This process will strengthen the attachment of the corneal flaps to the skirt, prevent extrusion of the skirt, leakage of aqueous and transmission of infectious agents into the anterior chamber.

The net-type skirt shown in Fig. 2 or the rings and ribs in Fig. 1 with large openings will allow free movement of fluids and nutrients between the two flaps of the cornea, thus preventing development of aseptic necrosis and melting of the peripheral cornea resulting in keratoprosthesis extrusion. The large openings in the skirt will also be used as a base for suturing keratoprosthesis to the limbus.

The keratoprosthesis operation is simple, practical and can be performed by most ophthalmic surgeons.

The removal of debris and damaged tissues within the anterior chamber will reduce the prevalence of retroprosthetic membrane substantially.

### IMPROVED BINDING

The skirt of keratoprosthesis is made of polymethylmethacrylate or silicone is hydrophobic and non-binding. To improve and consolidate the binding between the keratoprosthesis and the host cornea the following novel systems are incorporated.
a) Both surfaces of the skirt will be coated with 1 or 2 thin layers of a hydrophilic polymer such as polyhydroxyethylmethacrylate (PHEMA) or polydimethylsiloxane (we may need to use two coating system because of the number of ingredients). The coating will be performed by dipping or spraying or by crosslinking.
b) The binding between the skirt and the host cornea will be further enhanced and consolidated by adding a synthetic tissue bio-adhesive such as fibronectin to the single coating or one of the two coatings layers of the skirt. Studies have shown that tissue bio-adhesives generate strong binding between polymer surfaces and surrounding tissues.

### PREVENTION OF INFECTION

Ocular infections associates with keratoprosthesis usually develop following introduction of one or more pathogens into the wound and anterior chamber of the eye. The source of infection may include: the eye of recipient (normal bacterial flora of the eye), hands of surgeon, contaminated surgical equipment and materials and internal sources (rare).

Most gram positive and gram negative bacteria may cause post-operative ocular infection. These pathogens may reside and proliferate at the interface between the keratoprosthesis and the cornea or within damaged corneal tissues surrounding the keratoprosthesis and subsequently spread into the anterior chamber, causing severe endophthalmitis and destruction of the eye.

The present invention involves the use of a novel and highly effective system for prevention of infection associated with keratoprosthesis. The system is based on our patented drug delivery insert devices for delivery of drugs at the target area continuously and at controlled level for several weeks, now modified for the prevention of infection for this type of surgical procedure. See for example WO-A-9501764.

For prevention of infection associated with the keratoprosthesis of the invention there is incorporated two antibiotics (e.g. a combination of a macrolide and a quinolone, for providing maximum effect on both gram positive and gram negative bacteria) and release modifiers into the single coating or one of the two coating layers on the skirt. The antibiotics will be released at a zero or near zero order for several weeks into the space between the skirt and adjacent corneal tissues, where they can effectively neutralise the invading pathogens and preventing them from causing infection. Alternatively, one or both antibiotics can be incorporated into the base material of the skirt before moulding.

### PREVENTION OF INFLAMMATORY RESPONSES AND ASEPTIC NECROSIS

Inflammatory responses generally occur in response to operation trauma and/or presence of the polymer (foreign body) or polymer particles shedding from the surface of the skirt. The inflammatory responses caused by the operation may be moderate or severe, but generally subside within a few weeks. The inflammatory responses related to the polymer may start as mild responses, but gradually become more severe and causing damage to tissues surrounding the prosthesis and aseptic necrosis. Both of these inflammatory responses respond well to treatment with steroids or non-steroid anti-inflammatory compounds.

To prevent or substantially reduce development of inflammatory responses against the keratoprosthesis of the invention there is incorporated a system for sustained, continuous and controlled release of steroids (e.g. prednisolone) or non-steroid anti-inflammatory drugs (e.g. sodium diclofenac) at a very low concentration to the tissues surrounding the skirt. This is being achieved by: (a) incorporating a selected anti-inflammatory drug into the single or the two coating layers of the skirt; or (b) incorporating into the base material of the skirt before moulding. The compound will be released at zero or near zero order for a few weeks at the junction between the skirt and corneal tissues, which will be absorbed into the surrounding corneal tissues.

### PREVENTION OF RETROPROSTHETIC MEMBRANE

Retroprosthetic membrane commonly develops following trauma by surgical procedures to already damaged tissues within the anterior chamber of the eye and/or as a response to the presence of a synthetic polymer. after implanting a keratoprosthesis, the traumatised tissues within the anterior chamber release fibrin into the anterior chamber. Subsequently, the fibrin coagulates forming a pseudomembrane. After a few days, inflammatory cells invade the pseudomembrane and new vessels develop in the membrane and converting the pseudomembrane into a thick and well established membrane within the anterior chamber, which can block the pupil. Development of retroprosthetic membrane within the anterior chamber of the eye will be prevented by sustained, continuous and controlled release of anti-coagulants including heparin or its derivatives. This will be achieved by releasing heparin into the anterior chamber of the eye by one or more of the following novel methods:
a) Coating the posterior surface of the visual section of the keratoprosthesis of the invention by a transparent and bio-erodible polymer containing heparin and release modifiers which will release the compounds by bio-erosion and/or osmosis at continuous and controlled level for several weeks;
b) Incorporating heparin into a coating of our novel preventive corneal shield described in the aforesaid sections; and
c) Incorporating heparin into the base material of the visual section before moulding.

### PREVENTION OF EPITHELIAL IN-GROWTH

In order to prevent possible development of this serious and devastating complication, the following techniques are adopted:
a) Closing the wound by a surgical glue which prevents migration of epithelial cells in the wound;
b) Incorporating medroxyprogestron or other active ingredient (e.g. a steroid which is shown to prevent epithelial in-growth) into a coating of the anterior surface of the visual section of the keratoprosthesis. The coating consisting of a transparent and bio-erodible polymer may contain medroxyprogestron or other active ingredient and release modifiers which can release the active ingredient at a continuous and controlled level for a month or longer;
c) Incorporating the drug into a coating of our preventive corneal shield which can release the drug continuously and at controlled level for several weeks (see below).

### PREVENTIVE CORNEAL SHIELD

A novel preventive corneal shield to enhance healing of the corneal wound and to assist in preventing development of complications is also adopted in the preferred embodiments.

The novel shield consist of a soft contact lens made of PMMA, silicone or other suitable polymers which will be coated on both sides by thin layers of a liquid silicone containing release modifiers and active ingredients. The coating of the concave side may contain medroxyprogestron (a drug known to prevent epithelial in-growth) or compounds with similar function and an anti-inflammatory drug, to be released continuously and at controlled levels for several weeks. The coating of the convex side may contain antibiotics (e.g. a macrolide and a quinolone) and other active ingredients (if required) to be released at continuous and controlled levels for several weeks. The coating can be achieved by dipping, spraying or crosslinking.

The application of the specifically designed shield may:
a) Protect the surgical wound and enhance its healing. Studies have shown that contact lenses and shields are highly effective in protecting corneal wound;
b) Cover solidified remnants of surgical glue applied to the wound in order to prevent palpebral conjunctiva from being scratched;
c) Control bacterial flora of the eye, hence assisting in prevention of infection;
d) Assist in control of inflammatory responses, hence in preventing associated complications;
e) Prevent development of epithelial in-growth;
f) Eliminate the need for frequent applications of eye drops, eye ointments, sub-conjunctival injections and oral drugs which are required after keratoprosthesis operation.

Instruments required for implanting keratoprosthesis of the invention will be similar to those required for corneal transplantation with the addition of a trephine with a graduated guard and an angled corneal dissector. It should be noted that the diameter of the trephine should be ½ of a millimetre smaller than the diameter of the visual section of selected keratoprosthesis in order to compensate for contraction of the host cornea after trephination.

### ADVANTAGES OF THE KERATOPROSTHESIS OF THE INVENTION

The keratoprosthesis of the invention contains the following novel physical, polymeric, surgical and chemical elements which are particularly designed to remedy problems of rejection and complications commonly associated with keratoprosthesis.

Keratoprosthesis is a flexible one-piece prosthesis similar to the human cornea. In our in vivo studies it is shown that the prosthesis appears as a normal cornea.

The keratoprosthesis is made of a single polymer and as such it is technically simple to manufacture.

The design of the skirt particularly its thinness and inclusion of holes and slots, coating with a hydrophilic polymer containing a binding agent and specific sutures will promote a strong binding between the skirt and the host cornea.

Incorporation of antibiotics, anti-inflammatory, anti-coagulation and other active ingredients into the coatings of the skirt, to be released at sustained, continuous and controlled level for several weeks are designed to prevent infection, inflammation, aseptic necrosis, retroprosthetic membrane and epithelial in-growth, which are the common causes of failure and rejection of keratoprosthesis.

The keratoprosthesis allows examination of anterior and posterior segments of the eye.

The keratoprosthesis allows measurement of intra-ocular pressure and detection of glaucoma.

The operation procedure for implanting the keratoprosthesis is simple and only mildly traumatic and as such:
a) It can be performed by ophthalmologists with average surgical skills in approximately 30 minutes.
b) It is less prone to cause retinal detachment, haemorrhage and other intra-ocular complications.

The various improvements and techniques described above do not all have to be used together. For example in some cases not all coatings need to be applied. Also the use of the corneal shield may not be necessary. The skilled person can chose which features are required in particular circumstances and will obtain the benefits from the chosen features. For instance, the skilled person may elect not to adopt some or all of the drugs described above, but may still use the bio-adhesives. The release modifiers will then be used as required.

Generally, the present invention relates to a keratoprosthesis or artificial cornea which is designed as a light weight disc resembling a human cornea. The keratoprosthesis is maintained in the eye after surgery and is preferably adapted to reduce complications after surgery such as infection.

## Claims

1. A keratoprosthesis comprising a lens surrounded by a skirt, where the keratoprosthesis is a one-part construction with the thickness of the lens being greater than the thickness of the skirt.

2. The keratoprosthesis according to claim 1, wherein:
(a) the lens is generally circular with a diameter of between 5mm and 9mm, preferably about 7mm; and/or the skirt comprises a generally circular ring around the lens with the ring having a width of between 1mm and 3mm, preferably about 2mm; and/or
(b) the lens has a thickness of between 0.5mm and 2mm, preferably about 1mm; and/or the skirt has a thickness of between 0.1mm and 0.4mm, preferably about 0.3mm; and/or
(c) wherein the keratoprosthesis is formed from a biocompatible material selected from the group consisting of: silicone elastomers; polyacrylic polymers; polymethacrylic polymers; PMMA including modified and/or crosslinked PMMA, HEMA; polyHEMA; mixtures of the aforementioned compounds.

3. The keratoprosthesis according to claim 1 or claim 2, wherein at least one surface of the skirt is coated with a polymer which polymer is hydrophilic and porous, for example polyhydroxyethylmethacrylate or polydimethyl siloxane, and optionally:
(a) wherein both surfaces of the skirt are coated with said polymer; and/or
(b) wherein two thin layers of the same or different polymers are coated consequently to the skirt; and/or
(c) wherein the coating further includes release modifiers; and/or
(d) wherein the coating further contains at least one active ingredient selected from the group consisting of: at least one antibiotic, an anti-inflammatory drug and a tissue bio-adhesive compound; and in which case further optionally wherein the at least one active ingredient is arranged to be released at a controlled and continuous level into the space between the skirt and a host cornea; and/or
(e) wherein a or the coating layer adjacent to the skirt contains antibiotics to be released at controlled levels into the space between the skirt and a host cornea; and in which case further optionally including an anti-inflammatory drug and/or a tissue adhesive compound (e.g. fibronectin) arranged to be released into the said space and wherein the anti-inflammatory drug may comprise a steroid e.g. prednisolone, and/or a non-steroid anti-inflammatory drug e.g. sodium diclofenac arranged to be released in a controlled rate into tissue surrounding the skirt; and/or
(f) wherein active ingredients and/or release modifiers are incorporated into the base material of the skirt.

4. The keratoprosthesis according to any one of the preceding claims, wherein a posterior surface of the lens is coated with a transparent and bio-erodible polymer containing an anticoagulant, e.g. heparin or derivatives thereof; and preferably a release modifier arranged to release the anticoagulant by bioerosion and/or osmosis at continuous and controlled levels e.g. for several weeks; and optionally the anticoagulant and/or release modifiers are incorporated into the base material of the lens.

5. The keratoprosthesis according to any one of the preceding claims, wherein an anterior surface of the lens is coated by an anterior lens coating including a compound for preventing epithelial in-growth in a controlled manner; and optionally wherein the compound for preventing epithelial in-growth is medroxyprogestron, and/or wherein the anterior lens coating includes a release modifier arranged to release the compound for preventing epithelial in-growth in a controlled manner e.g. over one month.

6. The keratoprosthesis according to any one of the preceding claims wherein:
(a) the skirt includes two rings joined together by a plurality of ribs thus defining slots between the two rings and adjacent pairs of ribs; and in which case optionally wherein the skirt is provided with four ribs, thus defining four slots, and for wherein some or each rib further comprises a hole e.g. a large hole of about 1mm in diameter; or
(b) the skirt includes a plurality of large holes of about 1mm diameter; and/or the skirt including a plurality of small holes of about 0.3 diameter.

7. A kit comprising a keratoprosthesis according to any one of the preceding claims and a corneal shield, wherein the corneal shield enhances healing of the corneal wound made in implanting the keratoprosthesis and/or reduces post-operative complications.

8. The kit of claim 7, wherein
(a) the corneal shield comprises a soft contact lens e.g. made from PMMA, silicone or other suitable polymer; and/or
(b) at least one side of the corneal shield is coated with a thin layer of a liquid silicone or other suitable polymer, which incorporates a release modifier and active ingredient; and in which case optionally wherein both surfaces of the corneal shield are coated with different coatings; and/or wherein the coating on the concave side of the shield includes a drug for preventing epithelial in-growth (e.g. medroxprogestron) with a release modifier and/or the coating on the convex side comprises an antibiotic (e.g. a macrolide or quinolone) and release agent.

9. A corneal shield as defined in claim 7 or claim 8.

10. A kit comprising a keratoprosthesis according to any one of claims 1 to 6 or the kit according to claims 7 or 8, further including a suture assembly, e.g. where only two types of sutures, e.g. one continuous and four/six anchoring sutures, are required to hold the keratoprosthesis in place when inserted in the eye of a patient.
